# EUROPEAN PATENT APPLICATION

(11) **EP 3 871 712 A1**
(43) Date of publication of application: **01.09.2021**
(21) Application number: 19875901.1
(22) Date of filing: 21.10.2019
(51) Int. Cl.: A61M 5/315, A61M 5/145

(54) **INJECTION SYSTEM, SYRINGE, AND GASKET**

(30) Priority: 24.10.2018 JP 2018200114
(71) Applicant: Circulus Inc., Tokyo 113-0033 (JP)
(72) Inventor: UEKI, Jun, Yokohama city, Kanagawa 232-0065 (JP); FUKIKOSHI, Yumiko, Tokyo 113-0033 (JP)
(74) Representative: Viering, Jentschura & Partner mbB Patent- und Rechtsanwälte
(86) International application number: PCT/JP2019/041352
(87) International publication number: WO 2020/085318

(57) **Abstract**

An injection system 1 has: a gasket 100 having a plurality of engaging claws 122, and a distal end portion 140 having an oval cross-sectional shape in which a length in a height direction of the cross-sectional shape is longer than a length in a width direction perpendicular to the height direction; a ram 110 to be engaged with the gasket 100; a cylinder 91 into which the gasket 100 is to be inserted; and an injection device 2 configured to move the ram 110 forward and injecting a chemical solution in the cylinder 91, wherein each of the engaging claws 122 includes an inner surface S which defines a hole H having an inlet with an enlarged diameter and inclined in a direction away from a perpendicular line P passing through a center of the hole H, and the engaging claws 122 are to be displaced between a widened position and a narrowed position, and wherein, in the plurality of engaging claws 122, two engaging claws 122 aligned in the height direction have the same shape and size, and two engaging claws 122 aligned in the width direction have the same shape and size.

## Description

### BACKGROUND OF THE INVENTION

The present invention relates to an injection system on which a syringe, into which a chemical solution is loaded, is mounted.

### DESCRIPTION OF THE RELATED ART

Conventionally, as a ram and a gasket used in a syringe for injecting a chemical solution, for example, Patent Literature 1 describes a plunger having an expandable and shrinkable portion and a syringe having a first inner diameter and a second inner diameter smaller than the first inner diameter. An end of a plunger shaft is inserted into the space of the plunger. When the plunger moves forward in the syringe until it reaches the second inner diameter, the expandable and shrinkable portion shrinks. This causes a tab of the plunger (gasket) to engage with a groove of a plunger shaft (ram).

### CITATION LIST

### PATENT LITERATURE

Patent Literature 1: JP2014-111185A

### SUMMARY OF THE INVENTION

### TECHNICAL PROBLEM

In Patent Literature 1, the injection device is not been sufficiently miniaturized.

### SOLUTION TO PROBLEM

In order to overcome the above-described problems, one example of the present invention is an injection system comprising: a gasket having a plurality of engaging claws, and a distal end portion having an oval cross-sectional shape in which a length in a height direction of the cross-sectional shape is longer than a length in a width direction perpendicular to the height direction; a ram to be engaged with the gasket; a cylinder into which the gasket is to be inserted; and an injection device configured to move the ram forward and injecting a chemical solution in the cylinder, wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and wherein, in the plurality of engaging claws, two engaging claws aligned in the height direction have the same shape and size, and two engaging claws aligned in the width direction have the same shape and size.

Another example of the present invention is a syringe comprising: a gasket having a plurality of engaging claws, and a distal end portion having an oval cross-sectional shape in which a length in a height direction of the cross-sectional shape is longer than a length in a width direction perpendicular to the height direction; and a cylinder into which the gasket is to be inserted, wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and wherein, in the plurality of engaging claws, two engaging claws aligned in the height direction have the same shape and size, and two engaging claws aligned in the width direction have the same shape and size.

Still another example of the present invention is a gasket comprising: a plurality of engaging claws, and a distal end portion having an oval cross-sectional shape in which a length in a height direction of the cross-sectional shape is longer than a length in a width direction perpendicular to the height direction, wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and wherein, in the plurality of engaging claws, two engaging claws aligned in the height direction have the same shape and size, and two engaging claws aligned in the width direction have the same shape and size.

Further features of the present invention will become apparent from the following description of embodiments illustrated exemplarily with reference to the accompanying drawing.

### BRIEF DESCRIPTION OF THE DRAWINGS

Fig. 1 is a schematic perspective view of an injection head.
Fig. 2 is a schematic perspective view of the injection head.
Fig. 3 is a schematic perspective view of an adapter.
Fig. 4 is a schematic exploded perspective view of a syringe.
Fig. 5 is a schematic rear view of the syringe.
Fig. 6 is a schematic cross-sectional view of a gasket according to a first embodiment.
Fig. 7 is a schematic perspective view of the gasket viewed from a rear side.
Fig. 8 is a schematic cross-sectional view useful to describe the coupling between a ram and the gasket.
Fig. 9 is a schematic cross-sectional view useful to describe the coupling between the ram and the gasket.
Fig. 10 is a schematic cross-sectional view of a gasket according to a first embodiment.

### DESCRIPTION OF THE EMBODIMENTS

Now, exemplary embodiments for carrying out the present invention will be described in detail with reference to the drawings. It should be noted, however, that dimensions, materials, shapes, and relative positions between components described in the following embodiments are arbitrary and can be changed depending on the configuration or various conditions of the device to which the present invention is applied. Also, unless otherwise mentioned, the scope of the present invention is not limited to the embodiments specifically described below. Incidentally, in the following description, the front side ("front") corresponds to the distal end side of a syringe, and the opposite side corresponds to the rear side ("rear").

### First Embodiment

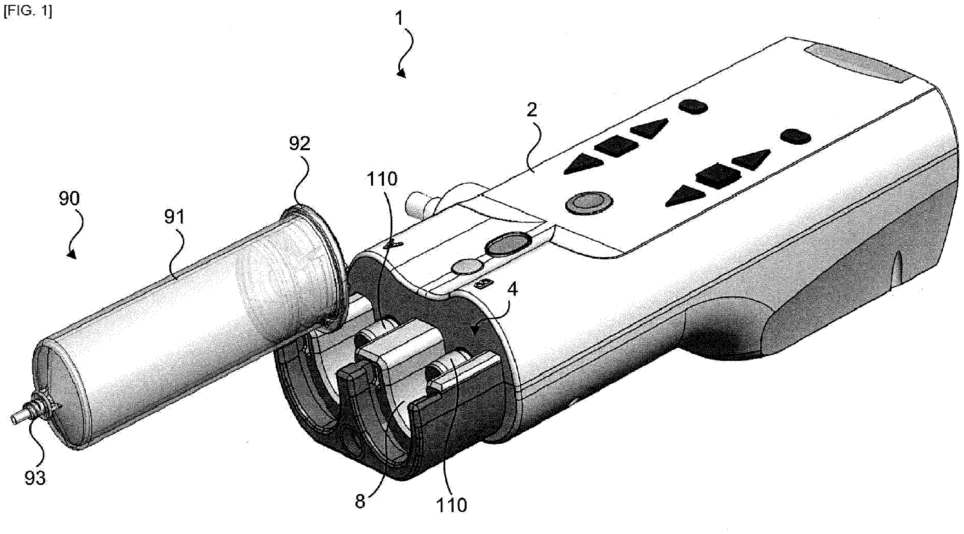

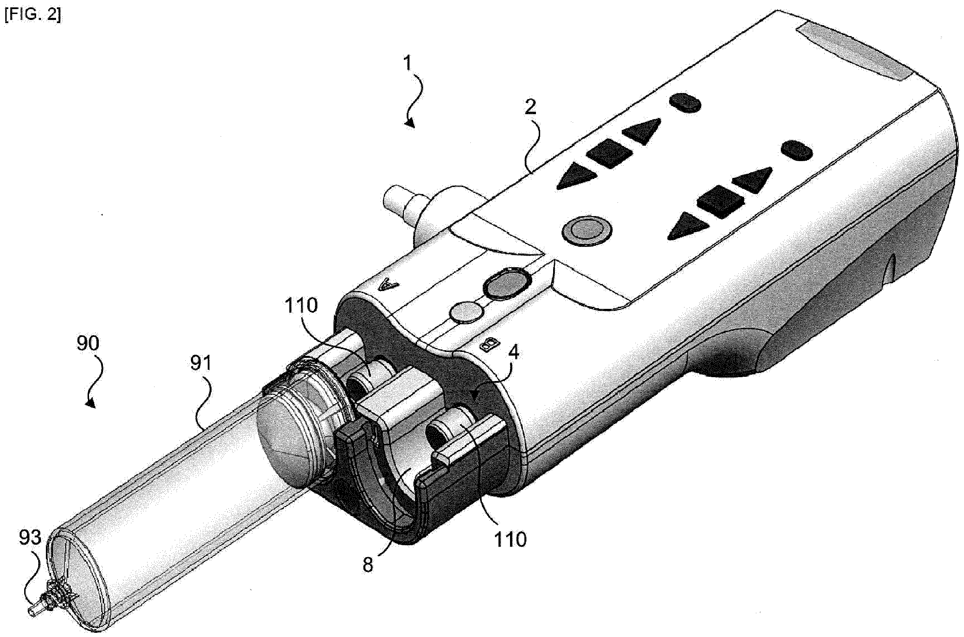

Fig. 1 and Fig. 2 are schematic perspective views of an injection system 1 for injecting a chemical solution. Fig. 1 shows the injection system 1 before mounting a syringe 90, and Fig. 2 shows the injection system 1 after mounting the syringe 90. As shown in Fig. 1, the injection system 1 includes an injection head 2 as one example of an injection device. The injection head 2 is configured to cause rams 110 to move forward to inject the chemical solution in a cylinder 91 of a syringe 90.

The injection system 1 is wired or wirelessly connected to an imaging device (not shown). At the time of injection of the chemical solution and photographing of images, various data are transmitted and received between the imaging device and the injection system 1. The imaging device is, for example, an MRI (Magnetic Resonance Imaging) device, a CT (Computed Tomography) device, an angio imaging device, a PET (Positron Emission Tomography) device, a SPECT (Single Photon Emission Computed Tomography) device, a CT angio device, an MR angio device, an ultrasonic diagnostic device or a vessel imaging device.

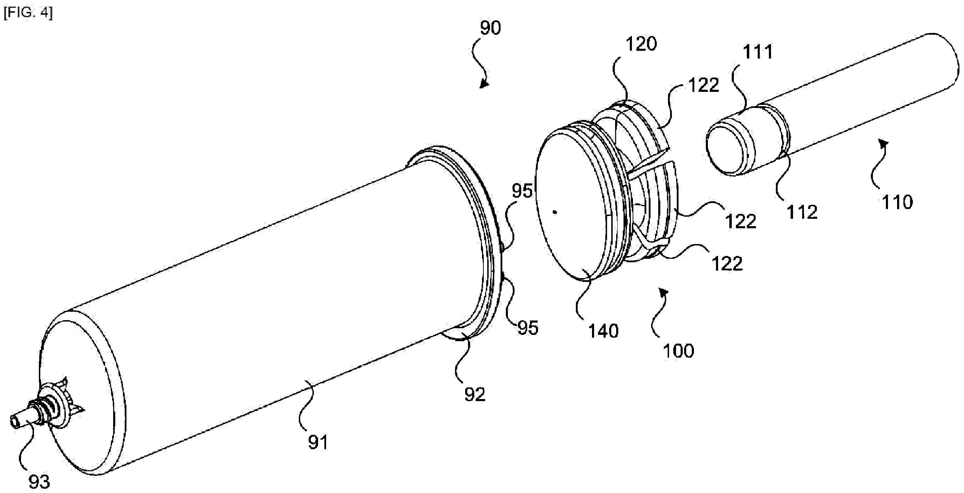

The injection head 2 of the injection system 1 is provided with pressing parts 4 for pushing gaskets 100 (Fig. 4) to be inserted into the respective syringes 90. Each of the pressing parts 4 is controlled by a control unit (not shown) such that the pressing part 4 presses and moves the gasket 100 forward in the syringe 90 in order to deliver the chemical solution from the syringe 90. Further, the pressing part 4 is provided with a drive mechanism (not shown) and a ram 110 which is connected to the drive mechanism. Specifically, the control unit controls a motor in the injection head 2 such that the ram 110 moves forward when the motor is rotating in a forward direction and the ram 110 moves backward when the motor is rotating in a reverse direction. Further, the injection head 2 may include a reading unit that reads a data carrier such as an RFID or a bar code provided on the syringe 90.

Each drive mechanism of the pressing parts 4 includes a transmission mechanism connected to the shaft of the motor, a ball screw shaft connected to the transmission mechanism, a ball screw nut attached to the ball screw shaft, and an actuator connected to the ball screw nut. The transmission mechanism has a pinion gear connected to the shaft and a screw gear connected to the ball screw shaft. The transmission mechanism transmits the rotation from the motor to the ball screw shaft. Therefore, the rotation of the shaft of the motor is transmitted to the ball screw shaft through the pinion gear and the screw gear. Thus, the ball screw shaft rotates in accordance with the transmitted rotation. The ball screw nut slides in the forward direction or the backward direction with the rotation of the ball screw shaft. As the ball screw nut slides, the ram 110 of the pressing part 4 moves forward or backward.

Further, the injection system 1 includes a console having a touch panel as a display unit for displaying an injection status of the chemical solution, and a control device (both not shown) having a control unit and a power supply. The console and the injection head 2 can be wired or wirelessly connected to each other. Additionally, a remote controller, such as a hand switch, may be wired or wirelessly connected to the console. The remote controller may also be used to start or stop the injection of the chemical solution. Incidentally, the injection head 2 and the control device can be integrally configured with a caster stand (not shown). Alternatively, the injection head 2 and control device may be provided separately and mounted on the caster stand.

Data of the operation pattern (injection protocol) and data of the chemical solution are stored in the control device in advance. When injecting a chemical solution into a patient, an operator operates the touch panel on the console to enter an injection speed, an injection volume and an injection time, as well as the patient's physical data such as body weight, height, body surface area, heart rate and cardiac output together with the data of the type of chemical solution. Then, the control device calculates an optimum injection condition according to the entered data and the data stored in advance. Thereafter, the control device determines an amount of chemical solution to be injected into the patient and the injection protocol based on the calculated injection condition.

In addition, upon determining the amount of chemical solution and the injection protocol, the control device displays predetermined data or graphs on the touch panel of the console. This allows the operator to see the displayed data or graphs. The data of the operation pattern (injection protocol) and the data of the chemical solution can be entered from an external storage medium. Alternatively, the injection head 2 is provided with a head display, and the control device displays predetermined data or graphs on the head display. If the head display is a touch panel, the operator may set injection conditions and the like from the head display. Further, the head display may be built in the injection head 2 or may be attached to the side of the injection head 2.

When injecting the chemical solution, the operator turns on the power of the injection head 2 and mounts the syringe 90 on the injection head 2 as shown in Fig. 2. Subsequently, the operator presses the injection button displayed on the touch panel. When the injection button is pressed, the control unit sends a forward-rotation signal as a drive voltage to the motor. In response to the forward-rotation signal, the shaft of the motor rotates in a forward direction and the ram 110 of the pressing part 4 moves forward. Thereafter, when the injection is completed and the syringe 90 is removed, the control unit sends a reverse-rotation signal as a drive voltage to the motor in order to cause the ram 110 to move backward. In response to the reverse-rotation signal, the shaft of the motor rotates in a reverse direction and the ram 110 moves backward.

If the injection head 2 is provided with an operation panel, the operator may press an injection button on the operation panel. The operator may press a button on a hand switch to initiate the injection. Alternatively, the operator may turn on the power of the injection head 2 after mounting the syringe 90.

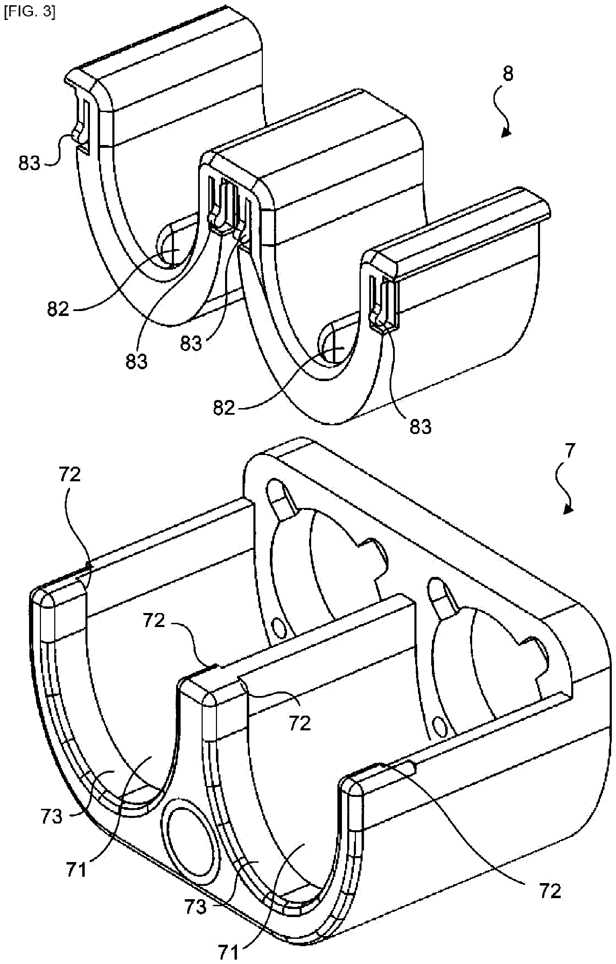

Fig. 3 is a schematic perspective view illustrating an adapter 8 for mounting the syringes 90 on the injection head 2. As shown in Fig. 3, the adapter 8 includes two grooves each having a substantially U-shaped cross-sectional shape and can be removably mounted to a holder 7 of the injection head 2. By way of example, the holder 7 has receiving portions 71 that form a shape complementary to the outer shape of the adapter 8. The operator attaches the adapter 8 to the holder 7 by inserting the adapter 8 into the receiving portions 71. Furthermore, the adapter 8 may be integrally formed with the holder 7.

In the holder 7, stepped portions 72, which protrude inward, are formed. Each stepped portion 72 defines a groove between the adapter 8 and the holder 7, which forms a clearance for receiving the flange 92 of the syringe 90 (Fig. 4). In addition, the holder 7 has two holding portions 73 each having a substantially U-shaped cross-sectional shape so as to hold the rear end portion of the syringe 90. In other words, the syringe 90 has a substantially oval cross-sectional shape, and the holding portion 73 of the holder 7 has a shape complementary to the outer shape of the syringe 90. As a result, the width of the syringe 90 in the horizontal direction orthogonal to the vertical direction of the syringe 90 decreases, so that the width of the holding portion 73 in the horizontal direction decreases. As a result, the width of the injection head 2 in the horizontal direction can be reduced, and the size of the injection head 2 can be reduced. That is, since the width of the syringe 90 is reduced as compared with a case in which the syringe 90 has a circular cross-sectional shape, the injection head 2 can be miniaturized. If the small injection head 2 is used, the injection system 1 can be installed in a CT examination room with a smaller space. In particular, in the injection head 2 in which the two syringes 90 can be mounted, the effect of miniaturization is significant. Alternatively, the holder 7 may be configured so that one or three or more syringes 90 may be mounted. In this case, the number of the holding portions 73 may be equal to the number of the syringes 90, which can be mounted on the holder 7.

In addition, a pair of engaging portions 83 are formed on the front surface of the adapter 8 and are positioned so that each holding portion 73 is disposed therebetween. In an example, each of the engaging portions 83 includes a straight portion extending downward and a protruding portion formed at the lower end of the straight portion. The engaging portion 83 has elasticity, and is deformed by being pushed by one of projections 95 formed on the rear surface of the cylinder 91 (Fig. 4). Specifically, the engaging portion 83 is displaced rearward when the engaging portion 83 is pushed by the projection 95. Thereafter, the engaging portion 83 is displaced forward by the elasticity thereof and returns to the original position thereof. Thus, the protruding portion of the engaging portion 83 passes over the projection 95 and is positioned above the projection 95. As a result, since the engaging portions 83 and the projections 95 are into contact with each other, upward movement of the syringe 90 is restricted by the engaging portions 83.

Furthermore, when the projection 95 passes over the engaging portion 83, the operator can obtain a click feeling. This allows the operator to perceive that the syringe 90 was deployed in the correct position. Alternatively, only one engaging portion 83 may be formed on one side of the holding portion 73, or three or more engaging portions 83 may be formed on both sides of the holding portion 73. Incidentally, the engagement portion 83 may have any other configuration as long as it has a protruding portion configured to be able to advance and retreat. For example, the engaging portion 83 may be composed of an elastic body protruding forward and a cover member covering the elastic body.

In addition, holes 82 are formed in the adapter 8 for allowing a liquid such as a chemical solution to escape to the outside of the injection head 2. Specifically, when the liquid is spilled onto the adapter 8, the liquid can escape to the outside through the holes 82. Accordingly, openings (not shown) corresponding to the holes 82 are also formed in the holder 7. Furthermore, the adapter 8 may be configured such that the syringes 90 are mounted thereon. In this case, grooves each receiving the flange 92 and holding portions each holding the syringe 90 may be formed in the adapter 8. Furthermore, the adapter 8 may be omitted and grooves for receiving the flanges 92 may be formed in the holder 7.

Fig. 4 is a schematic exploded perspective view of the syringe 90 showing the syringe 90 viewed from above and from the front side. As shown in Fig. 4, the syringe 90 includes a cylinder 91 having a distal end portion 93 and a flange 92 that is to be inserted between the adapter 8 and the holder 7. The syringe 90 also includes a gasket 100 that is slidable within the cylinder 91. The gasket 100 is inserted into the cylinder 91 such that the outer surface of the inserted gasket 100 is in contact with the inner surface of the cylinder 91. The gasket 100 engages with a front end portion 111 of the ram 110 and slides within the cylinder 91 by movement of the ram 110.

The gasket 100 includes a seal member 140 as the distal end portion thereof that has an oval cross-sectional shape. In the cross section of the seal member 140, the length in the height direction is longer than the length in the width direction perpendicular to the height direction. The gasket 100 also includes a sucker 120 having a plurality of split engaging claws 122, and the front end of the sucker 120 is inserted into the seal member 140. The sucker 120 is made of an elastic resin such as POM (polyacetal resin), and can be manufactured by molding. The seal member 140 is made of, for example, butyl rubber, and can be manufactured by molding.

The gasket 100 is coupled to the ram 110 by which the engaging claws 122 engage with the front end portion 111 of the ram 110. When the motor rotates in a forward direction, with the engaging claws 122 being coupled to the ram 110, the pressing part 4 pushes the ram 110 in the forward direction. As the ram 110 and the gasket 100 move forward, the chemical solution in the cylinder 91 is pushed out through the distal end portion 93. As a result, the chemical solution is injected into the patient's body through an extension tube, or the like connected to the distal end portion 93. After the injection of the chemical solution, when the motor rotates in the reverse direction, the pressing part 4 pulls the ram 110 in the retracting direction such that the gasket 100 retracts. Furthermore, the engaging claws 122 may have a groove into which an O-ring is fitted. In this case, by fitting the O-ring, it is possible to regulate the engaging claws 122 from widening. The distal end 93 can be connected to the extension tube via a locking connector, a luer lock, a push-in connector, or the like.

The cylinder 91 into which the gasket 100 is inserted has a cross-sectional shape complementary to the gasket 100. That is, the cylinder 91 has a substantially oval cross-sectional shape in which the length in the height direction of the cross-sectional shape is longer than the length in the width direction perpendicular to the height direction. Accordingly, when the rear end portion of the syringe 90 is held by the holding portion 73 of the holder 7, both side surfaces of the rear end portion are in contact with the inner surface of the holding portion 73. Thus, the syringe 90 is prevented from rotating relative to the holder 7. For example, at the time of the injection of the chemical solution, the gasket 100 is pushed into the cylinder 91 by the ram 110 that moves forward while rotating. At this time, the rotational force is transmitted to the syringe 90 via the gasket 100. However, since both side surfaces of the rear end portion are in contact with the inner surface of the holding portion 73, the syringe 90 is prevented from rotating.

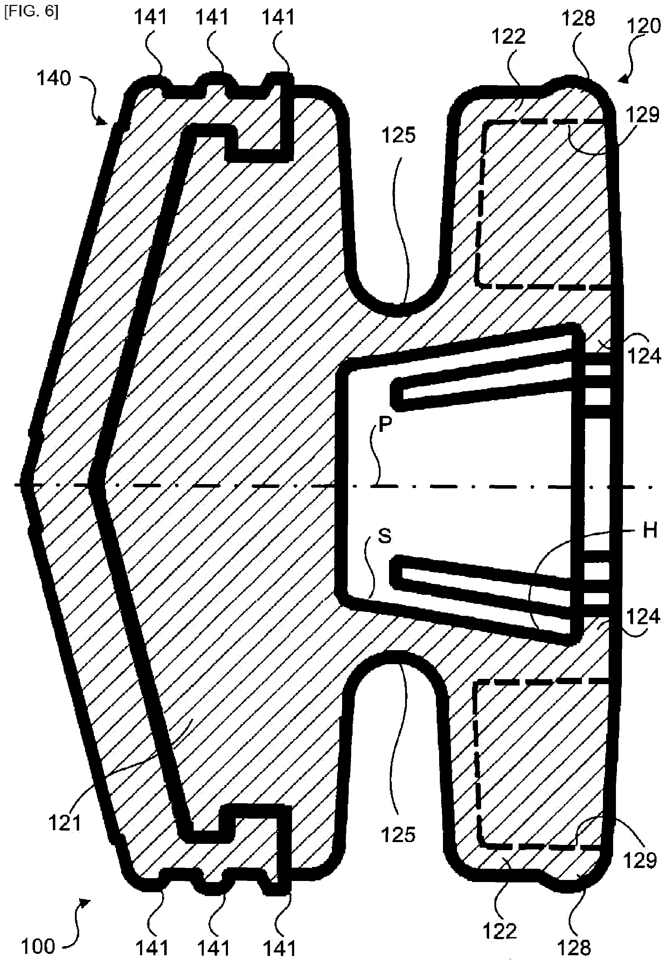

The ram 110 is made of, for example, stainless steel or aluminum, and can be manufactured by welding a solid substantially cylindrical front end portion 111 to a hollow pipe. Alternatively, the ram 110 may be manufactured by screwing a solid front end portion 111 into a hollow pipe. Furthermore, the front end portion 111 may be made of a material other than stainless steel or aluminum, for example, a material having a hardness higher than that of the gasket 100. At the front end portion 111, an annular engaging groove 112 extending in the circumferential direction is formed. By inserting the protrusions 124 of the engaging claws 122 (Fig. 6) into the engaging groove 112, the gasket 100 and the ram 110 are engaged with each other.

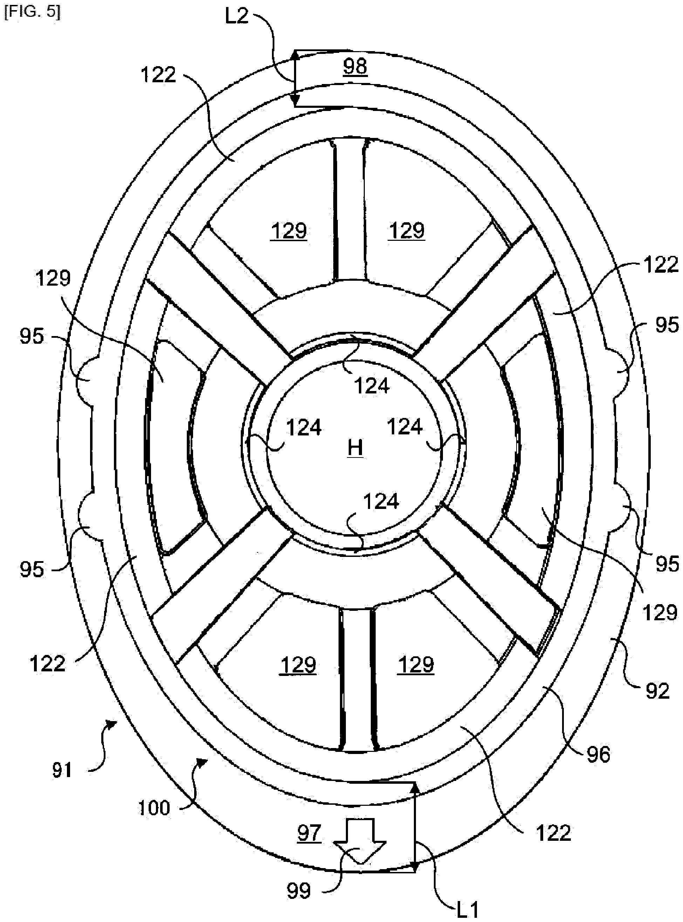

Fig. 5 is a schematic rear view of the syringe 90 into which the gasket 100 is inserted. As shown in Fig. 5, the syringe 90 has projections 95. Two projections 95 are formed on each side of the cylinder 91, so that the syringe 90 has four projections 95. The projections 95 protrude laterally from an annular portion 96 protruding rearward from the flange 92. That is, the substantially ring-shaped annular portion 96 is formed in the flange 92, and the projections 95 are formed so as to project from the annular portion 96. When mounting the syringe 90, the projections 95 pass over the engaging portions 83 of the adapter 8. After the syringe 90 is mounted, since the engaging portions 83 abuts on the projections 95, upward movement of the syringe 90 is restricted. Alternatively, the projections 95 may be formed on only one side of a hole H formed at the center of the gasket 100, or three or more projections 95 may be formed around the hole H.

The flange 92 has a substantially oval outer shape. However, the length L1 of a first portion 97 located on the lower side in Fig. 5 in the height direction (in the vertical direction of Fig. 5) is longer than the length L2 of a second portion 98 located on the upper side in the height direction. That is, the length L1 of the first portion 97 extending upward from the cylinder 91 in the height direction is longer than the length L2 of the second portion 98 extending downward. This allows the operator to visually recognize the correct orientation and vertical direction of the syringe 90. Also, since the first portion 97 is long, the contact area between the holder 7 or the adapter 8 and the flange 92 is increased. Thus, since the syringe 90 can be stably held, the syringe 90 can withstand a higher injection pressure. Furthermore, the first portion 97 of the flange 92 is given an arrow 99 that indicates the insertion direction.

Alternatively, the upwardly extending second portion 98 may be longer than the downwardly extending first portion 97. Also in this case, the operator can visually recognize the correct orientation and insertion direction of the syringe 90. Furthermore, if the second portion 98 extending upward is long, and the flange 92 is inserted into the groove between the holder 7 and the adapter 8, it is possible to prevent the second portion 98 from being erroneously inserted downward. That is, since the depth of the groove corresponds to the length of the shorter first portion 97, if the flange 92 is inserted with incorrect orientation, the second portion 98 comes into contact with the bottom of the groove before the projections 95 pass over the engaging portions 83. Therefore, the operator cannot obtain the click feeling and can recognize that the direction is incorrect. This allows the operator to modify the syringe 90 in the correct orientation for mounting.

In the gasket 100, two engaging claws 122 aligned in the height direction, which is one direction, have the same shape and size. Furthermore, two engaging claws 122 aligned in the width direction, which is another direction perpendicular to the height direction, have the same shape and size. As a result, the amount of displacement of the engaging claws 122 in the height direction can be substantially matched with the amount of displacement of the engaging claws 122 in the width direction orthogonal to the height direction. That is, since the gasket 100 has a substantially oval outer shape, in a case in which the shape and size of the plurality of engaging claws 122 are uniformed, the amount of displacement of the engaging claws 122 in the height direction is different from the amount of displacement of the engaging claws 122 in the width direction. In order to substantially equalize the amounts of displacement of all engaging claws 122, the shape and size of the engaging claws 122 aligned in the height direction are different from those of the engaging claws 122 aligned in the width direction.

That is to say, in the gasket 100 having a substantially oval outer shape, two engaging claws 122 aligned in the height direction are formed to have the same size. In the gasket 100, two engaging claws 122 aligned in the width direction are also formed to have the same size. In addition, a substantially circular hole H, into which the front end portion 111 is inserted, is formed at the central portion surrounded by the four engaging claws 122. Therefore, the height of the engaging claws 122 aligned in the height direction is longer than the height of the engaging claws 122 aligned in the width direction. In addition, a substantially fan-shaped recess 129 is formed in each of the engaging claws 122. Furthermore, the protrusions 124 for engaging with the engaging groove 112 of the ram 110 are formed at the periphery of the hole H. The protrusions 124 face the engaging groove 112 when the ram 110 is inserted so that the end face of the front end portion 111 is brought into contact with the bottom of the hole H.

Fig. 6 is a schematic cross-sectional view of the gasket 100 along the front-back and height directions. The sucker 120 has a substantially disk-shaped insertion portion 121, and a plurality of intersecting ribs (not shown) are formed in the insertion portion 121. An annular groove 125 is formed between the insertion portion 121 and the engaging claws 122. The seal member 140 has annular projections 141 formed on the outer periphery thereof. The annular projections 141 are in contact with the inner surface of the cylinder 91, so that the cylinder 91 is sealed. Incidentally, in Fig. 2, three annular projections 141 are formed, but two, one or four or more annular projections may be formed. Furthermore, a space for accommodating the insertion portion 121 is formed in the seal member 140, and the rear end portion of the seal member 140 protrudes toward the space.

Substantially fan-shaped recesses 129 are formed in the engaging claws 122 of the sucker 120. The recesses 129 can reduce the thickness of the engaging claws 122. Therefore, the engaging claws 122 are more deformable than insertion portion 121. The portion at which the annular groove 125 is formed is thinner than the portion at which the engaging claws 122 are formed and the insertion portion 121. Thus, the annular groove 125 can be a starting point of deformation of the engaging claws 122. The bottom of the annular groove 125 has a substantially semicircular shape in cross section. Alternatively, the bottom of the annular groove 125 may have a substantially trapezoidal or substantially triangular cross-sectional shape so as to be tapered inwardly.

The hole H of the sucker 120 is narrowed toward the bottom, and when the ram 110 is inserted into the hole H, the end face of the front end portion 111 comes into contact with the bottom of the hole H. In the state in which the end face of the front end portion 111 is in contact with the bottom of the hole H, a clearance is produced between the outer surface of the front end portion 111 and the inner surface S of the hole H. That is, the inner surface S of the hole H is inclined toward the center of the bottom. Therefore, the front end portion 111 inserted into the hole H is guided so as to slide on the inner surface S inclined toward the center of the bottom. As a result, the front end portion 111 can be aligned with the center of the hole H.

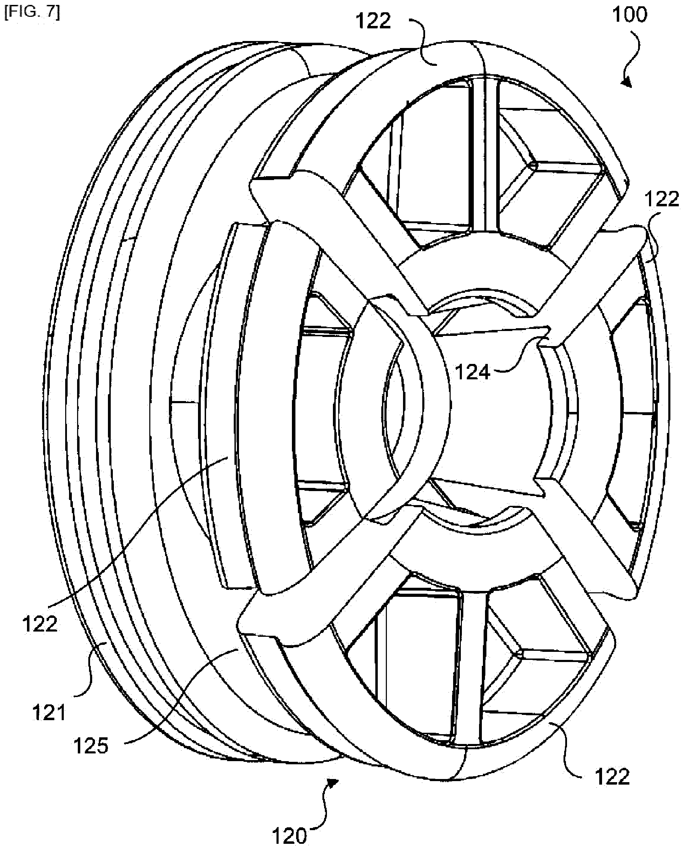

Fig. 7 is a schematic perspective view of a gasket 100 viewed from the rear. As shown in Fig. 7, the sucker 120 of the gasket 100 includes the multiple engaging claws 122, for example, four engaging claws 122 each having a substantially fan-shape. Alternatively, the sucker 120 may include two, three, or five or more engaging claws 122. Furthermore, a clearance is formed between adjacent engaging claws 122, and when the gasket 100 is moved forward within the cylinder 91, the engaging claws 122 are displaced so that adjacent engaging claws 122 approach each other. Since all the clearances have the same length and width, it is possible to restrict change in positions of the respective engaging claws 122 relative to the ram 110 when displacing the engaging claws 122. That is to say, as the gasket 100 is moved forward, the respective engaging claws 122 are displaced so as to approach each other by the same distance.

Each engaging claw 122 is provided with a protrusion 124 that engages with the engaging groove 112 of the ram 110. The protrusion 124 protrudes toward the center of the sucker 120. Furthermore, the distal end of the protrusion 124 is rounded so as to be easily inserted into the engaging groove 112. In Fig. 7, only one protrusion 124 is denoted by the reference numeral. However, the projections 124 are formed on all the four engaging claws 122, respectively.

Furthermore, the annular groove 125 is formed between the insertion portion 121 and the engaging claws 122. In the portion in which the annular groove 125 is formed, the engaging claws 122 are connected to the insertion portion 121. To facilitate the displacement or change in shape of the engaging claws 122, the portion in which the annular groove 125 is formed is thinner than the portion in which the protrusions 124 are formed. A hole H surrounded by the protrusions 124 is formed in the sucker 120, and the ram 110 is inserted into the hole H so as to engage with the engaging claws 122.

### Coupling of the ram 110 and the gasket 100

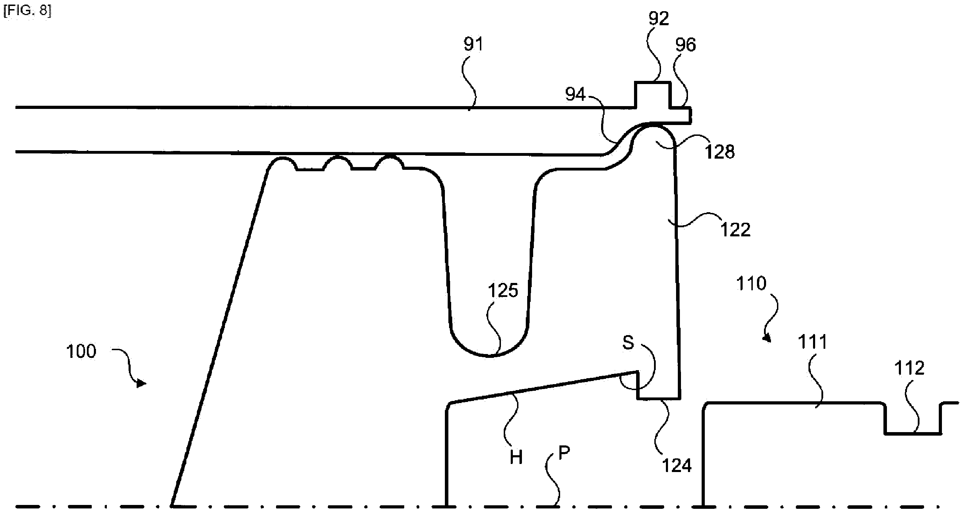

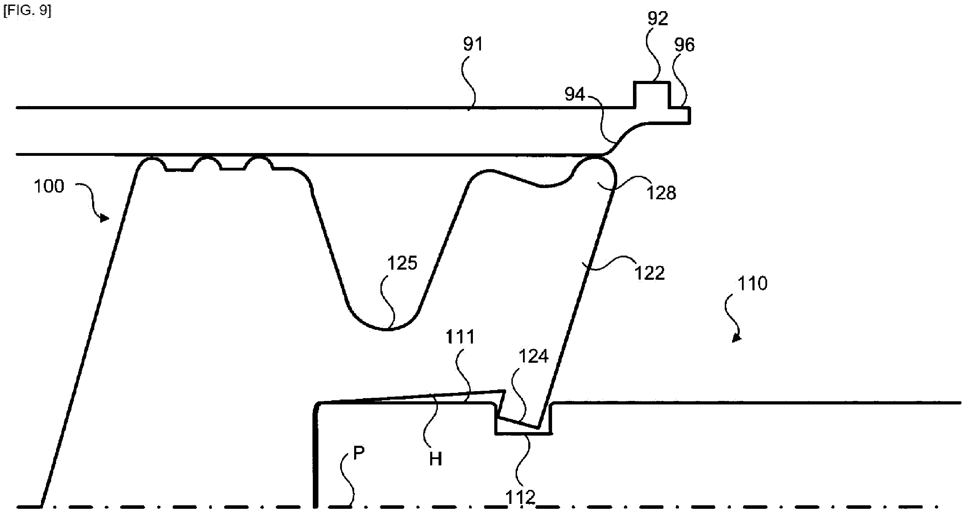

Referring to Figs. 8 and 9, the connection of the ram 110 and the gasket 100 will be described. Fig. 8 is a schematic cross-sectional view of the syringe 90 prior to insertion of the ram 110 into the hole H of the sucker 120. Fig. 9 is a schematic cross-sectional view of the syringe 90 with ram 110 advancing the gasket 100. Furthermore, Figs. 8 and 9 show a cross section along the longitudinal direction including the central axis of the cylinder 91. For convenience of description, in Figs. 8 and 9, the sucker 120 and the seal member 140 are integrally shown. Furthermore, Figs. 8 and 9 show only the upper halves of the ram 110 and the gasket 100 in the height direction. The lower portions of the ram 110 and gasket 100 in the height direction, except for the flange 92, have substantially the same shape as the upper portions.

As shown in Fig. 8, the gasket 100 has the hole H having an inlet of which the diameter is enlarged. That is, in the hole H, the inlet through which the ram 110 is inserted has a longer inner diameter as compared to the bottom to which the end face of the front end portion 111 of the ram 110 presses. The gasket 100 has engaging claws 122 that displaces between widened positions (Fig. 8) and narrowed positions (Fig. 9). The engaging claws 122 includes an inner surface S that defines the hole H and that is inclined in a direction away from a perpendicular line P passing through the center of the hole H (bottom).

Each engaging claw 122 has a ridge 128 protruding in directions away from the perpendicular line P passing through the center of the hole H. Furthermore, an enlarged diameter portion 94 for receiving the ridges 128 is formed in the rear end portion of the cylinder 91. That is, the inner diameter of the rear end portion of the cylinder 91 is increased as compared with the inner diameter of other portions. When the gasket 100 is assembled to the cylinder 91, the ridges 128 are received in the enlarged diameter portion 94, and then the entirety of the gasket 100 is accommodated in the cylinder 91. Alternatively, the rear portions of the engaging claws 122 may protrude outward from the cylinder 91. The engaging claws 122 have the protrusions 124 protruding inward of the hole H. The ram 110 has the annular engaging groove 112, which engages with the protrusions 124. Incidentally, the enlarged diameter portion 94 may be a stair-like stepped surface, or an inclined surface or a curved surface that is narrowed toward the center of the cylinder 91.

As shown in Fig. 9, when the ram 110 is inserted into the hole H, the end face of the front end portion 111 comes into contact with the bottom of the hole H. Then, when the ram 110 pushes the gasket 100, the gasket 100 moves forward in the cylinder 91. When the gasket 100 is moved forward, the ridges 128 of the engaging claws 122 come into contact with the enlarged diameter portion 94 of the cylinder 91. Then, when the ridges 128 are moved forward and passe through the enlarged diameter portion 94, the engaging claws 122 are displaced toward the perpendicular line P. At this time, the engaging claws 122 are deformed such that the center of the bottom of the annular groove 125 serves as a starting point of deformation.

Thus, even if the front end portion 111 is inserted at a position biased with respect to the perpendicular line P in the hole H, the front end portion 111 is displaced such that the central axis of the front end portion 111 is coincident with the perpendicular line P. That is, the front end portion 111 is pushed by a portion corresponding to the bottom of the annular groove 125 and is displaced toward the center of the hole H. Therefore, it is possible to restrain the central axis of the ram 110 from being inclined with respect to the gasket 100. As shown in Fig. 9, after the ridges 128 pass the enlarged diameter portion 94, the engaging claws 122 are narrowed. The protrusions 124 of the engaging claws 122 in the narrowed positions enter the engaging groove 112, and the protrusions 124 are engaged with the engaging groove 112. As a result, the gasket 100 is connected to the ram 110. Furthermore, with the displacement of the engaging claws 122, the annular groove 125 is deformed so as to spread.

Thereafter, when the gasket 100 is moved forward in the cylinder 91, the seal member 140 presses the chemical solution in the cylinder 91. As a result, the chemical solution is pushed out from the distal end portion 93 and injected into the body of the patient through the extension tube or the like. After the injection of the chemical solution, the ram 110 is retracted, and the gasket 100 connected to the ram 110 is also retracted. Then, when the ram 110 and the gasket 100 are retracted to the position, at which the ridges 128 are received, in the enlarged diameter portion 94, the engaging claws 122 extend outward so that the protrusions 124 are disengaged from the engaging groove 112.

Thus, the protrusions 124 are disengaged from the engaging groove 112. That is, when the ram 110 and the gasket 100 are retracted to the position in which the engaging claws 122 is displaced to the widened positions, the protrusions 124 exit from the engaging groove 112. Furthermore, with the displacement of the engaging claws 122, the annular groove 125 narrows so as to return to the original shape. When the ram 110 is further retracted, the gasket 100 remains in the position shown in Fig. 8 by the frictional force between the seal member 140 and the cylinder 91. As a result, the ram 110 leaves the gasket 100 and retracts to the position before insertion shown in Fig. 8.

If the ram 110 and the gasket 100 according to the first embodiment are used, the engaging claws 122 are deformed such that the center of the bottom of the annular groove 125 serves as a starting point of deformation. Therefore, the multiple engaging claws 122 are uniformly displaced toward the perpendicular line P of the sucker 120. Thus, when the ram 110 is detached from the gasket 100, it is possible to restrict the front end portion 111 from being biased with respect to the perpendicular line P. Accordingly, the engaging groove 112 of the front end portion 111 can be prevented from being hooked with a protrusion 124.

Furthermore, if the ram 110 and the gasket 100 according to the first embodiment are used, and the gasket 100 and the ram 110 are connected, rattling of the ram 110 relative to the gasket 100 can be reduced. Furthermore, since the gasket 100 and the ram 110 are directly connected to each other, the distance between the syringe 90 and the pressing part 4 can be shortened. Therefore, the size of the injection head 2 in the injection system 1 can be reduced.

Furthermore, if the syringe 90 of the first embodiment is used, the size in the width direction can be reduced as compared with a case in which the syringe has a substantially circular cross-sectional shape. That is, the width of the syringe 90 can be narrowed, or the size of the syringe 90 can be reduced. In addition, by increasing the size of the syringe 90 in the height direction so that the syringe 90 has a substantially oval cross-sectional shape, the syringe 90 can have the same capacity and a smaller size. As a result, the injection head 2 can be reduced in size and weight. In particular, in a case in which an actuator having a rotation transmission mechanism accommodated in a case is incorporated in the frame of the injection head 2, the injection head 2 can be reduced in size synergistically. Furthermore, by increasing the size in the height direction, the contact area to the holding portion 73 is increased. Thus, the syringe 90 can be stably fixed.

Furthermore, if the syringe 90 of the first embodiment is used, the syringe 90 can be held by the holding portion 73 simply by an action of inserting the syringe 90 into the holding portion 73. Thus, the syringe 90 can be more easily mounted by omitting an action of rotating the syringe 90 for attaching it to the injection head 2. Furthermore, since the width of the syringe 90 is reduced, the syringe 90 can be easily grasped even by an operator having a small hand. Furthermore, since both side surfaces in the width direction are flat surfaces, rolling of the syringe 90 can be prevented when it is placed on a flat surface of a table or the like.

Furthermore, since the contact distance and the contact area to the holding portion 73 increase in the height direction (depth direction), the chemical solution can be stably injected. That is, both side surfaces (inner surfaces) of the syringe 90 in the width direction function as fixed guides for guiding the gasket 100. Therefore, during sliding the gasket 100, it is possible to restrict the gasket 100 or the suction element 120 from being rotated relative to the cylinder 91 due to sliding resistance at the time of injection. Thus, distortion of the gasket 100 that causes the backflow can be restricted, so that the gasket 100 can be stably moved forward and backward by the ram 110. Furthermore, since the flange 92 can be small, it is possible to reduce the cost by reducing the material for manufacturing the cylinder 91.

Furthermore, since the diameter of the syringe 90 in the height direction is different from that in the width direction, the operator can visually recognize the orientation of the syringe 90 and the direction of attaching and detaching. Furthermore, by attaching the scales to the upper surface of the cylinder 91, the portion to which the scales are attached is positioned above the holder 7 or the adapter 8. This makes the scales easier for the operator to see. Furthermore, when air is mixed into the cylinder 91, the air is accumulated in the upper space in the cylinder 91 that is farther from the distal end portion 93, so that the air is restricted from flowing out to the patient side.

### Second Embodiment

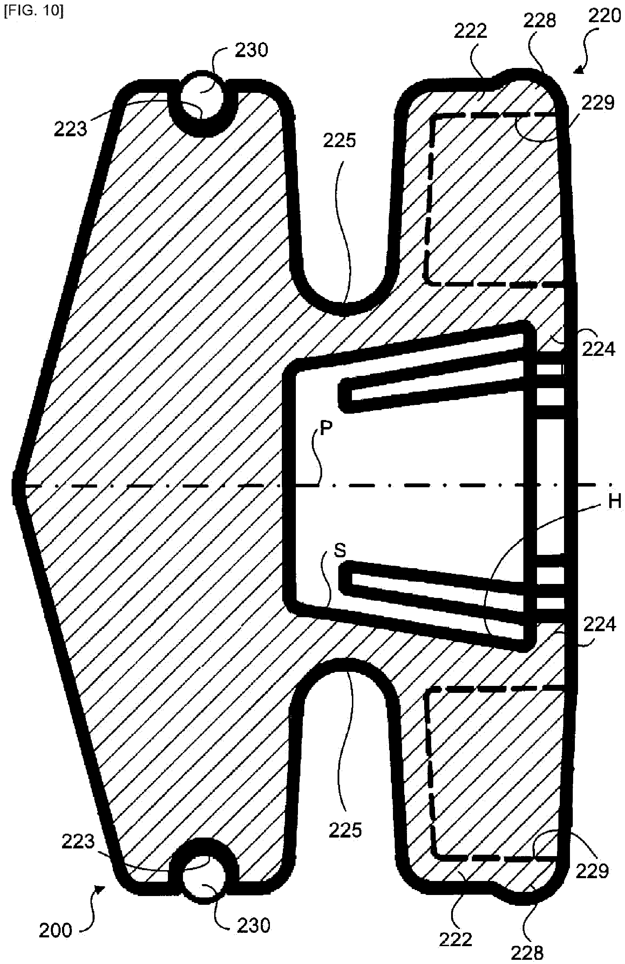

In the first embodiment, the gasket 100 includes a seal member 140 and a sucker 120. The second embodiment is different from the first embodiment in that the gasket 200 includes a sucker 220 and an O-ring 230. Hereinafter, the second embodiment will be described with reference to Fig. 10, but in the description of the second embodiment, differences from the first embodiment will be described whereas those components which are already described in the first embodiment are given the same reference numerals and the description thereof will be omitted. Unless otherwise mentioned, the components denoted by the same reference numerals operate and function in the substantially same manner and achieve the substantially same advantages.

Fig. 10 is a schematic cross-sectional view of a gasket 200 along the front-back and height directions. The gasket 200 includes the sucker 220 having a distal end portion having an oval cross-sectional shape, and the O-ring 230 fitted into the sucker 220. The O-ring 230 is received in an annular O-ring groove 223 formed in the peripheral surface of the sucker 220. The O-ring 230 is in contact with the inner surface of the cylinder 91, so that the cylinder 91 is sealed. Alternatively, there may be two or more O-rings 230, and in this case, two or more O-ring grooves 223 may be formed.

The sucker 220 has a plurality of engaging claws 222 and an annular groove 225 formed between the engaging claws 222 and the O-ring groove 223. To facilitate displacement of the engaging claws 222, the portion in which the annular groove 225 is formed is thinner than other portions. A substantially fan-shaped recess 229 is formed in each of the engaging claws 222. The recesses 229 can reduce the thickness of the engaging claws 222. Therefore, the engaging claws 222 are more easily deformed.

A hole H having a shape narrowed toward the bottom is formed at the central portion surrounded by the engaging claws 222. When the ram 110 is inserted into the hole H, the end face of the front end portion 111 of the ram 110 comes into contact with the bottom of the hole H. In the state in which the end face of the front end portion 111 is in contact with the bottom of the hole H, a clearance is produced between the outer surface of the front end portion 111 and the inner surface S of the hole H. Therefore, the front end portion 111 inserted into the hole H is guided so as to slide on the inner surface S inclined toward the center of the bottom. As a result, the front end portion 111 can be aligned with the center of the hole H. A clearance is formed between adjacent engaging claws 222, and when the gasket 200 is moved forward within the cylinder 91, the engaging claws 222 are displaced so that adjacent engaging claws 222 approach each other. All the clearances between the engaging claws 222 have the same length. Thus, when displacing the engaging claws 222, it is possible to restrict change in positions of the respective engaging claws 222 relative to the ram 110.

Each engaging claw 222 is provided with a protrusion 224 that engages with the engaging groove 112 of the ram 110. The distal end of the protrusion 224 is rounded so as to be easily inserted into the engaging groove 112 of the ram 110. The gasket 200 of the second embodiment, similarly to the first embodiment, includes an inner surface S that defines a hole H having an inlet of which the diameter is enlarged, and an annular groove 225 that serves as a starting point of deformation. Furthermore, each engaging claw 222 has ridge 228 protruding outward. When the gasket 200 is assembled to the cylinder 91, the ridges 228 are received in the enlarged diameter portion 94 of the cylinder 91, and then the entirety of the gasket 200 is accommodated in the cylinder 91.

When the ram 110 is connected to the gasket 200, the ram 110 is inserted into the hole H, and the end face of the front end portion 111 of the ram 110 comes into contact with the bottom of the hole H. Then, when the ram 110 pushes the gasket 200, the gasket 200 moves forward in the cylinder 91. When the gasket 200 is moved forward, the ridges 228 of the engaging claws 222 come into contact with the enlarged diameter portion 94. Then, when the ridges 128 are moved forward and passe through the enlarged diameter portion 94, the engaging claws 222 are displaced toward the perpendicular line P. At this time, the engaging claws 222 are deformed such that the center of the bottom of the annular groove 225 serves as a starting point of deformation.

Thus, even if the front end portion 111 is inserted at a position biased with respect to the perpendicular line P in the hole H, the front end portion 111 is displaced such that the central axis of the front end portion 111 is coincident with the perpendicular line P. Therefore, it is possible to restrain the central axis of the front end portion 111 from being inclined with respect to the gasket 200. After the ridges 228 pass the enlarged diameter portion 94, the engaging claws 222 are narrowed. The protrusions 224 of the engaging claws 222 in the narrowed positions enter the engaging groove 112 of the ram 110, and the protrusions 224 are engaged with the engaging groove 112. As a result, the gasket 200 is connected to the ram 110. Furthermore, with the displacement of the engaging claws 222, the annular groove 225 is deformed so as to spread.

Thereafter, when the gasket 200 is moved forward in the cylinder 91, the sucker 220 presses the chemical solution in the cylinder 91. As a result, the chemical solution is pushed out from the distal end portion 93. After the injection of the chemical solution, the ram 110 is retracted, and the gasket 200 connected to the ram 110 is also retracted. Then, when the ram 110 and the gasket 100 are retracted to the position, at which the ridges 228 are received in the enlarged diameter portion 94, the engaging claws 222 extend outward so that the protrusions 224 of the displaced engaging claws 222 are disengaged from the engaging groove 112.

Furthermore, with the displacement of the engaging claws 222, the annular groove 225 narrows so as to return to the original shape. When the ram 110 is further retracted, the gasket 200 remains in the cylinder 91 by the frictional force between the O-ring 230 and the cylinder 91. As a result, the ram 110 leaves the gasket 200 and retracts to the position before insertion.

Also, if the ram 110 and the gasket 200 according to the second embodiment are used, the engaging claws 222 are deformed such that the center of the bottom of the annular groove 225 serves as a starting point of deformation. Therefore, the multiple engaging claws 222 are uniformly displaced toward the perpendicular line P of the sucker 220. Thus, when the ram 110 is detached from the gasket 200, it is possible to restrict the front end portion 111 from being biased with respect to the perpendicular line P. Accordingly, the engaging groove 112 of the front end portion 111 can be prevented from being hooked with a protrusion 224.

Furthermore, if the ram 110 and the gasket 200 according to the second embodiment are used, rattling of the ram 110 relative to the gasket 200 can be reduced when the gasket 200 and the ram 110 are connected. Furthermore, since the gasket 200 and the ram 110 are directly connected to each other, the distance between the syringe 90 and the pressing part 4 can be shortened. Therefore, the size of the injection head 2 in the injection system 1 can be reduced. Furthermore, since the size in the width direction can be reduced, the syringe 90 can have the same capacity and a smaller size. As a result, the injection head 2 can be reduced in size and weight.

While the present invention has been described with reference to the respective embodiments, the present invention is not limited to the above-described embodiments. Inventions modified to the extent that they are not contrary to the present invention, and inventions equivalent to the present invention are also included in the present invention. Further, each embodiment and modifications described above can be appropriately combined within the scope not contrary to the present invention.

For example, notches or holes may be formed in the engaging claws 122 and 222. In this case, since the portions in which the notch or hole are formed are deformed, the claws 122 and 222 are easily displaced. In addition, the inner surface S of the gasket 100 or 200 may be divided into two surfaces of which inclination is different. Furthermore, the inner surface S may constitute a continuous inclined surface or curved surface. Furthermore, the gasket 100 or 200 may have an egg-like outer shape in which the length in the height direction is longer than the length in the width direction perpendicular to the height direction. In this case, the cylinder 91 may have a shape complementary to the egg-like outer shape.

Furthermore, the syringe 90 into which the chemical solution is loaded may be a prefilled syringe. The chemical solution may also be manually filled into the syringe 90, or may be filled into the syringe 90 by the injection head 2 or a loading device. The syringe 90 may be provided with a data carrier, such as a RFID or bar code. In the data carrier, information about the loaded chemical solution is recorded. The injection system 1 can read the recorded information from the data carrier through the injection head 2 and control the injection amount of the chemical solution. For example, the control device may calculate an optimum injection amount per body weight based on the retrieved information (iodine amount) of the chemical solution and display it on the touch panel of the console. Incidentally, when a chemical solution is filled into empty syringes 90, the chemical solution may be sucked from a large-capacity bottle provided with a data carrier. In this case, it is possible to monitor the amount of chemical solution in the large-capacity bottle and to inject the chemical solution multiple times. Furthermore, the information of the chemical solution recorded in the data carrier of the large-volume bottle may be retrieved and written into RFID tags or the like of empty syringes 90. Furthermore, the retrieved information may be used for setting in the injection head 2.

Furthermore, notches may be formed in certain portions of the outer periphery of the flange 92. Specifically, the flange 92 may have two arcuate portions to become an arcuate shape with respect to the center axis of the syringe 90, and two flat portions formed between the arcuate portions and facing each other. In this case, the adapter 8 may be provided with engagement portions for engaging with the notches, e.g., locking claws, convex portions, or latches.

Furthermore, instead of forming the enlarged diameter portion 94 in the cylinder 91, a skirt portion extending rearward from the flange 92 may be formed. The inner surface of the skirt portion is inclined, and receives the ridges 128 of the engaging claws 122 so that the engaging claws 122 are in the widened positions. Therefore, the inner dimensions of the skirt portion are set to match the outer dimensions of the engaging claws 122 in the widened state. That is, the skirt portion has a shape that narrows toward the distal end portion 93. As a result, the engaging claw 122 of the gasket 100 inserted into the skirt portion are not displaced to the narrowed positions. Alternatively, even if the engaging claws 122 are slightly displaced, the distance between the opposing protrusions 124 can be maintained such that the ram 110 can be inserted.

In this case, after inserting the front end portion 111 of the ram 110, the gasket 100 moves forward in the cylinder 91 as the ram 110 pushes the gasket 100. As the gasket 100 moves forward, the ridges 128 of the engaging claw 122 pass through the skirt portion and comes into contact with the inner surface of the cylinder 91. Along with the forward movement of the gasket, the reaction force from the inner surface of the cylinder 91 causes the engaging claws 122 to be displaced toward the center of the hole H of the sucker 120.

Furthermore, instead of forming the enlarged diameter portion 94 to the cylinder 91, the rear portion of the gasket 100 may project outwardly from the cylinder 91. In this case, when the ram 110 pushes the gasket 100, the gasket 100 is moved forward in the cylinder 91. As the gasket 100 moves forward, the ridges 128 of the engaging claws 122 pass over the rear end of the cylinder 91 and comes into contact with the inner surface of the cylinder 91. When the ridges 128 pass over the rear end of the cylinder 91, the reaction force from the inner surface of the cylinder 91 displaces the engaging claws 122 toward the perpendicular line P of the sucker 120.

Part or all of the above-described embodiments may be described as in the following supplementary note, but not limited thereto.

### Supplementary Note 1

An injection system comprising:
a gasket having a plurality of engaging claws respectively includes an inner surface which defines a hole having an inlet with an enlarged diameter and a ridge protruding in a direction away from a perpendicular line passing through a center of the hole, and the engaging claws are to be displaced between a widened position and a narrowed position;
a ram to be inserted into the hole so as to be engaged with the engaging claws;
a cylinder into which the gasket is to be inserted, and which contacts with the ridge of each engaging claw of the gasket after insertion; and
an injection device configured to move the ram forward and injecting a chemical solution in the cylinder,
wherein an annular groove which serves as a starting point of deformation of the engaging claws is formed on the gasket.

### Supplementary Note 2

The injection system according to Supplementary Note 1, wherein an enlarged diameter portion for receiving the ridges is formed in a rear end portion of the cylinder.

This application claims a priority from Japanese Patent Application No. 2018-200114, filed October 24, 2018, the entirety of which is incorporated herein by reference.

### REFERENCE SINGS LIST

1: Injection system, 2: Injection device, 91: Cylinder, 100: Gasket, 110: Ram, 200: Gasket

## Claims

1. An injection system comprising:
a gasket having a plurality of engaging claws, and a distal end portion having an oval cross-sectional shape in which a length in a height direction of the cross-sectional shape is longer than a length in a width direction perpendicular to the height direction;
a ram to be engaged with the gasket;
a cylinder into which the gasket is to be inserted; and
an injection device configured to move the ram forward and injecting a chemical solution in the cylinder,
wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and
wherein, in the plurality of engaging claws, two engaging claws aligned in the height direction have the same shape and size, and two engaging claws aligned in the width direction have the same shape and size.

2. The injection system according to claim 1, wherein each of the engaging claws includes a ridge protruding in directions away from the perpendicular line, and
an enlarged diameter portion for receiving the ridges is formed in a rear end portion of the cylinder.

3. The injection system according to claim 1 or 2, wherein an annular groove which serves as a starting point of deformation of the engaging claws is formed on the gasket.

4. A syringe comprising:
a gasket having a plurality of engaging claws, and a distal end portion having an oval cross-sectional shape in which a length in a height direction of the cross-sectional shape is longer than a length in a width direction perpendicular to the height direction; and
a cylinder into which the gasket is to be inserted,
wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and
wherein, in the plurality of engaging claws, two engaging claws aligned in the height direction have the same shape and size, and two engaging claws aligned in the width direction have the same shape and size.

5. A gasket comprising:
a plurality of engaging claws, and a distal end portion having an oval cross-sectional shape in which a length in a height direction of the cross-sectional shape is longer than a length in a width direction perpendicular to the height direction,
wherein each of the engaging claws includes an inner surface which defines a hole having an inlet with an enlarged diameter and inclined in a direction away from a perpendicular line passing through a center of the hole, and the engaging claws are to be displaced between a widened position and a narrowed position, and
wherein, in the plurality of engaging claws, two engaging claws aligned in the height direction have the same shape and size, and two engaging claws aligned in the width direction have the same shape and size.
